# EUROPEAN PATENT SPECIFICATION

(11) **EP 1 833 376 B1**
(45) Date of publication and mention of the grant of the patent: **12.09.2012**
(21) Application number: 05818482.1
(22) Date of filing: 15.12.2005
(51) Int. Cl.: A61B 1/32, A61B 17/02

(54) **IMPROVEMENTS IN OR RELATING TO SPECULA**
VERBESSERUNGEN VON ODER IN VERBINDUNG MIT SPEKULA
PERFECTIONNEMENTS APPORTES OU AYANT TRAIT A DES SPECULUMS

(30) Priority: 15.12.2004 GB 0427506; 22.08.2005 GB 0517103
(43) Date of publication of application: 19.09.2007
(73) Proprietor: George, Samuel, Weybridge, Surrey KT13 9SB (GB)
(72) Inventor: George, Samuel, Weybridge, Surrey KT13 9SB (GB)
(74) Representative: Cummings, Sean Patrick
(86) International application number: PCT/GB2005/004858
(87) International publication number: WO 2006/064247

(56) References cited:
- WO-A-00/69325
- WO-A-92/21279
- WO-A-98/11818
- US-A- 6 096 046

## Description

The present invention relates to a speculum, and in particular to a vaginal speculum for enabling examination and treatment of the vaginal walls and cervix.

As is generally known, vaginal specula are used by physicians for dilating the opening of the vaginal cavity in order that the vaginal walls and cervix may be more easily visible and accessible for examination, diagnosis and treatment by surgery or otherwise.

Standard bivalve specula typically comprise two blades (an upper blade and a lower blade), joined near their proximal ends by a fixed hinge. At least one of the blades includes a handle, depending from the proximal end of the blade, for the physician to hold. The co-operating proximal end portions of the blades define a proximal aperture, through which aperture the physician may observe and access the vaginal cavity and cervix with instruments for inspection, investigation or surgery. Commonly the handle comprises two operating levers that can be moved relative to one another to open or close the blades.

In use, the speculum is positioned in the vaginal canal so that the upper blade is adjacent the top of the vaginal canal and the lower blade is adjacent the bottom of the vaginal canal. The blades are then splayed apart by operation of the levers to dilate the vaginal canal by pressing apart its top and bottom. In view of the fixed hinge, the dilation of the vaginal canal is greatest at the distal ends of the blades and decreases towards their proximal ends.

Typically, specula are also provided with a locking mechanism for locking the blades in position against vaginal wall muscle contraction once opened to a desired extent. A typical locking mechanism comprises a threaded rod joined by a pivot to one operating lever and a nut in threaded engagement with the rod and which can be tightened against the other operating lever. It will be appreciated that locking the position of the open blades requires both hands and can be an awkward manoeuvre.

Although the bivalve speculum is effective in widening the cervical end of the vaginal canal by splaying apart the blades, the access to the vaginal canal is determined by the diameter of the introitus of the vagina, and hence by the proximal aperture which normally cannot be widened any further. It will be appreciated that better access may be required during some treatments such as surgical procedures or in order to use certain medical instruments.

Accordingly, mobile-hinged specula, such as Graves' speculum, exist in which the upper and lower blades are not directly joined together so that the upper blade and the lower blade can be moved apart without the distal ends of the blades splaying apart. However, although this is a more adaptable speculum than the conventional fixed-hinge type, it is more complicated and more time-consuming to operate because the blade separation, blade flaring and locking operations all involve separate actions and are difficult or impossible to perform with one hand. This protracted routine is also not desirable from the point of view of the patient who would prefer the examination or treatment to be quick and to require generally less manipulation of the speculum.

A further problem associated with most speculum designs is that the handle or handles of the specula are normally at an acute angle, or at a right angle, to the blades. This inevitably results in the physician's hands and fingers being in contact with, or in close proximity to, the patient's genitalia, upper thighs and buttocks during a gynaecological procedure, which may be distressing to the patient and lead to accusations of impropriety against the physician.

Known speculum designs are ineffective in the case of patients having lax vaginal walls which prolapse and protrude inwardly between the open blades of the speculum in use, thus obstructing the physician's view and access and hindering procedures such as cervical smear-taking and treatment such as electrosurgery.

To overcome vaginal side wall prolapse, four-bladed specula are known in the art in which two additional blades are provided for supporting and pressing back the vaginal side walls during examination and treatment. Specula of this type are described in US 5 868 668 and US 6 024 696. In some cases, however, the additional blades must be manually operated by the physician when the speculum is in position, in addition to operation of the upper and lower blades as aforesaid. This inevitably requires the use and co-ordination of both hands which is cumbersome and awkward for the physician and also delays the procedure while both hands of the physician are occupied in operating the speculum. It is also unpleasant for the patient who may feel both of the physician's hands come into contact with, or be in proximity to, her genitalia, upper thighs and buttocks.

These problems have been partially overcome by the applicant's earlier invention as disclosed in WO 00/69325, which describes a speculum having two blades joined near their proximal ends by a floating hinge or pivot which allows the blades to move apart to widen the proximal opening without the blades necessarily splaying apart. Advantageously, the speculum includes an operating mechanism which allows one-handed operation. Also described are supplementary blades attached to the lower blade whose lateral splaying can also be controlled with one hand and which interact with a pin and a post provided on the primary blades upon squeezing together the operating levers. Further, the operating lever and handle are obtusely angled in a proximal direction with reference to the blades so that the physician's hands and fingers are spaced further away from the patient's body when operating the speculum.

Although the floating hinge allows some widening of the proximal opening and therefore access into the vaginal cavity, this widening is limited by the length of the floating hinge itself and so this speculum is still not appropriate for use with some surgical procedures where a yet wider access is required into the vaginal cavity.

Additionally, the supplementary blades and associated parts make it difficult to clean and sterilise the instrument. This is especially pertinent in light of the current fear of MRSA 'super-bug' infections in hospitals and clinics. To combat the spread of MRSA, it is now policy in most if not all hospitals to dispose of medical instruments after single use unless their construction allows for effective cleaning and sterilisation. Undoubtedly, this would mean that the speculum described in WO 00/69325, as well as other similar prior art instruments such as those disclosed in US 6 048 308 and WO 99/12466, would be treated as single-use instruments because of their construction and so disposed of after one use. However, the cost of manufacture of these complex instruments can be high. In addition, the design of such specula can necessitate the use of metals, with the associated fabrication and material costs being higher than if plastics could be used. It is apparent that the resulting wastage, both in terms of costs and materials, is significant. If instruments are to be disposable, one use only, they need to be as simple and inexpensive as possible but without losing functionality.

The invention aims to provide a vaginal speculum which overcomes or minimises the problems mentioned above.

The invention resides in a supplementary blade for a speculum, the speculum having a plurality of primary blades movable relative to each other for opening and closing, wherein the supplementary blade comprises an anchor portion attachable to the speculum and a blade portion movable with respect to the anchor portion, and the blade portion is shaped to interact with at least one of the primary blades to move into an extended position in response to opening of the primary blades. The supplementary blade has a concave inner face shaped to lie against a convex outer surface of one or more of the primary blades.

The supplementary blade may for example have at least one wing lying outside a plane containing the anchor portion. Preferably, the supplementary blade is resilient to return to a retracted position when the primary blades close.

Advantageously, lax vaginal side walls can be pushed back or supported by the supplementary blade in the extended position. This is in addition to the effect of the open primary blades of the speculum on the vaginal walls, and allows a practitioner to carry out observation or treatment of the vagina or cervix without hindrance. As the movement of the supplementary blade between the extended and the retracted positions results from the interaction between the blade portion of the supplementary blade and at least one of the primary blades, no additional levers or handles are required for its operation. This means that a physician can operate the speculum with one hand and use the other hand for taking samples and/or treatment if required, which causes less discomfort to the patient and a faster overall procedure than using a more complex speculum.

Preferably, the supplementary blade has a discontinuous lateral cross-section interrupted by two parallel creases and the cross-section includes two or more generally flat portions extending from each crease. The supplementary blade preferably also has a major portion tapering generally toward a free end opposed to the anchor portion, a minor portion tapering generally toward the anchor portion and a neck joining the anchor portion to the blade portion. The lateral cross-section of the supplementary blade comprises a spine and two wings lying to the same side of the spine and is symmetrical about the central longitudinal axis of the spine.

Conveniently, the supplementary blade is attachable to a speculum by means of a releasable fastening on the anchor portion, the releasable fastening preferably comprising a stud engageable within a hole in the speculum. This means that the supplementary blade need only be attached to the speculum when required or detached in circumstances when it is not required. Additionally, the releasable fastening on the anchor portion is such that the supplementary blade can be used with existing and conventional specula designs and so there is no need to adapt existing equipment. As the supplementary blades have such a simple construction and can be easily made from plastics, they are cheap to manufacture. The supplementary blades, or specula fitted with the supplementary blades, are therefore viable as disposable devices. Alternatively, the supplementary blades can be cleaned and sterilised separately from the speculum.

A further advantage of the supplementary blade is that its anchor portion comprises a fastening attachable to a speculum in two or more attachment positions, a first attachment position being laterally inward of a second attachment position. The stud has two or more waists each defining a respective attachment position. These two or more attachment positions means that the desired extension of the supplementary blade in the extended position can be controlled or chosen according to the patient and the situation.

The invention also extends to a speculum having a plurality of primary blades movable relative to each other for opening and closing, and being fitted with at least one supplementary blade, the supplementary blade being positioned to interact with at least one of the primary blades to move into an extended position in response to opening of the primary blades.

Another arrangement, not forming part of the present invention, includes a speculum having a plurality of primary blades movable relative to each other for opening and closing, wherein at least one of the blades comprises a means for attaching a supplementary blade thereto to increase the functional length of the at least one blade. Advantageously, the attachment means comprises an opening for receiving a portion of the supplementary blade to attach it to the at least one blade. The opening may comprise a channel for receiving a portion of the supplementary blade, the supplementary blade being movable within said channel to vary the functional length of the blade.

Another speculum, not forming part of the present invention, comprises first and second blades whose opening and closing is controlled by manipulating respective handles, wherein at least one of the handles comprises ratchet means engageable by the other of said handles.

Advantageously, the ratchet means is on a platform extending rearwardly from one of the handles and the other of the handles moves across the platform when the handles are manipulated in use. The platform may carry a series of protrusions, for example transverse ridges. Advantageously, the other of the handles has a free edge co-operable with the protrusions and, optionally, comprises two or more engagement means for engagement with the ratchet means.

Preferably, if two or more engagement means are provided, they are spaced along the handle and are selectable by moving the handles about a floating hinge to bring the ratchet means into co-operation with the selected one of the engagement means. Such an engagement means may be defined by an edge of an opening in a handle.

By virtue of these features, the speculum can be locked in the open blade position more easily than with the typical nut/screw locking mechanism of conventional specula. The physician can lock the blades of the speculum into position using only one hand which is more comfortable for the patient and speeds up the procedure.

A thumb rest may protrude from a free end of at least one of the handles. This assists the physician in operating the handles of the speculum and in locking the blades in position.

Also disclosed, but not forming part of the present invention, is a package comprising a wrapper encapsulating at least part of a medical instrument such as a speculum, the package containing lubricant between the wrapper and the instrument to lubricate at least an encapsulated part of the instrument before removal of the wrapper.

Advantageously, the incorporation of the lubricant facilitates the use of the medical instrument such as the insertion of the speculum into a cavity of the patient.

Advantageously, at least the encapsulated part of the instrument is pre-lubricated with lubricant before being encapsulated in the wrapper. This means that the pre-lubricated and encapsulated instrument can be a sterile unit, which is ready for the practitioner to use. Advantageously, the practitioner need not spend time smearing lubricant over the instrument with his fingers before use which might increase the chances of contamination.

Preferably, lubricant is localised within the wrapper, and is optionally confined to its location within the wrapper. The lubricant may be confined by a breachable barrier within the wrapper. The material of the barrier is suitably weaker than the material of the wrapper, such that, for example, the barrier can be breached by user pressure upon the exterior of the wrapper. Alternatively, the barrier can be breached under pressure from the instrument, or by a combination of user and instrument pressure.

Advantageously, lubricant is localised adjacent a distal end of the instrument within the wrapper. However, the wrapper is preferably flexible such that lubricant can be distributed over the instrument upon manipulation of the wrapper.

The wrapper preferably completely encapsulates the instrument but the wrapper may comprise a minor compartment containing lubricant and a major compartment for encapsulating at least part of the instrument.

Also disclosed, but not forming part of the present invention, is a method of lubricating a medical instrument before use, comprising supplying the instrument at least partially encapsulated in a wrapper with lubricant between the wrapper and the instrument, and manipulating the wrapper to distribute lubricant over the instrument before removal of the wrapper. The lubricant is preferably initially localised within the wrapper and is preferably confined by a breachable barrier within the wrapper. In this case, the method suitably comprises manipulating the wrapper and/or the instrument to breach the barrier for distribution of the lubricant over the instrument.

In order that this invention may be more readily understood, currently preferred embodiments will now be further described by way of example with reference to the accompanying drawings, in which:
Figure 1 is a plan view of (a) an inside face, and (b) an outside face of a supplementary blade according to the present invention;
Figure 2 is a cross-sectional view on line X-X' of the supplementary blade of Figure 1 when viewed from the proximal end;
Figure 3 is a cross-sectional view on line Y-Y' of the supplementary blade of Figure 1;
Figure 4 is a cross-sectional view on line Z-Z' of the supplementary blade of Figure 1;
Figure 5 is a top plan view of two supplementary blades of Figure 1 attached to a conventional speculum having an upper and a lower primary blade, when the primary blades of the speculum are in a closed position;
Figure 6 is a top plan view corresponding to Figure 5 but showing the splayed apart supplementary blades, when the primary blades of the speculum are in an open position;
Figure 7 is a side view of the supplementary blades and speculum arrangement of Figure 5, when the primary blades of the speculum are in the closed position;
Figure 8 is a side view of the supplementary blades and speculum arrangement of Figure 6, when the primary blades of the speculum are in the open position;
Figure 9 is a cross-sectional view through the line Z-Z' of the supplementary blade of Figure 1 when attached to the conventional speculum of Figure 5, when the primary blades of the speculum are in the closed position;
Figure 10 is a cross-sectional view through the line Z-Z' of the supplementary blade of Figure 1 when attached to the conventional speculum of Figure 5, when the primary blades of the speculum are in the open position;
Figure 11 is a perspective view of a modified speculum;
Figure 12 is a perspective view of the speculum of Figure 11 assembled with the supplementary blade of Figure 1;
Figure 13 is a perspective view of another speculum, having an upper blade and handle and a lower blade and handle;
Figure 14 is a perspective view of the lower blade and handle of the speculum of Figure 13;
Figure 15 is a perspective view of the speculum of Figure 13 assembled with the supplementary blade of Figure 1;
Figure 16 is a perspective view from the side of a variant of the upper blade and handle of the speculum of Figure 13;
Figure 17 is a perspective view from the side of an alternative variant of the speculum of Figure 13;
Figure 18 is a perspective view from the side of the speculum of Figure 17 assembled with the supplemental blade of Figure 1;
Figure 19 is a perspective view from the side of a variant of the upper blade and handle of the speculum of Figure 17; and
Figure 20 is a perspective view of a conventional speculum having a lubrication packet.

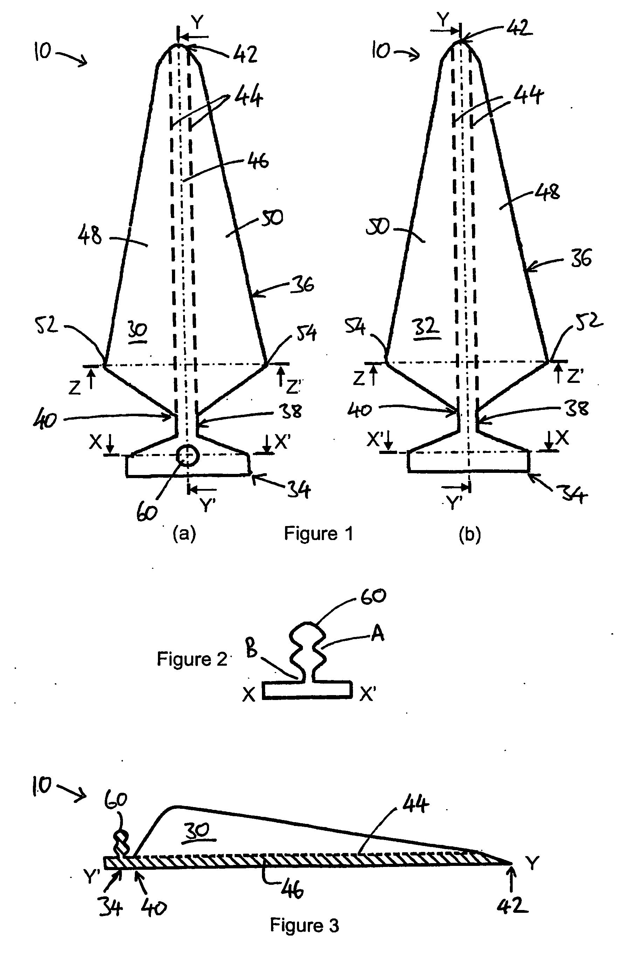

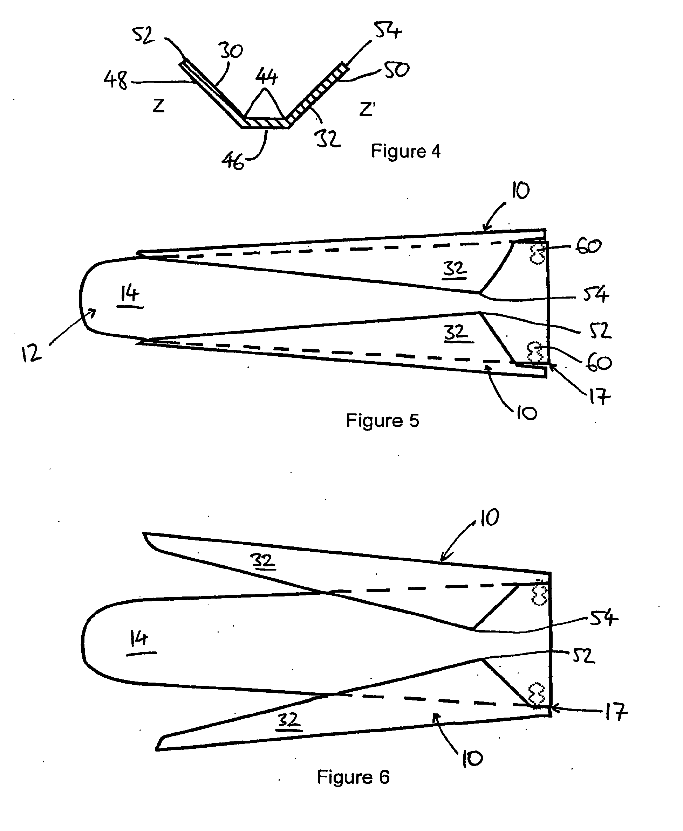

Referring initially to Figures 1 to 4, there is shown a supplementary blade 10 which can be attached or fixed to a conventional speculum 12 (shown in part in Figures 5 to 8) the conventional speculum 12 typically having two primary blades, an upper blade 14 and a lower blade 16, which are joined or co-operate near their proximal ends 17 by a hinge, pivot or fulcrum. Typically, the hinge 18 comprises two co-axial joints 20.

The co-operating proximal end portions 17 of the primary blades 14, 16 define a proximal aperture (not shown) through which the vaginal cavity of a patient can be accessed. In conventional manner, the primary blades 14, 16 and the hinge 18 are arranged so that the blades 14, 16 can be moved apart, about the joints 20, to splay apart the blades 14, 16 which pushes back the vaginal walls of a patient in use. Although not shown in these figures, the supplementary blade 10 of the present invention may also be used with a speculum in which the proximal ends of the primary blades are able to move apart to widen the proximal opening.

The supplementary blade 10 is intended to push back or support vaginal side walls in patients with lax vaginal walls, in other situations where there is a risk of prolapse of the vaginal walls, or where prolapse has occurred. The supplementary blade 10 has an inside face 30 and an outside face 32 and comprises an anchor portion 34, a blade portion 36 and a hinge portion 38. The length of the blade portion 36 is approximately three quarters of the length of a primary blade of a conventional speculum.

The blade portion 36 is generally trowel-shaped in that moving from its proximal end 40, it widens to a maximum width near its proximal end 40 and then tapers along its length towards its distal end 42. Further, the blade portion 36 has a concave inside face 30 whose shape complements the convex outside curvature of the two primary blades 14, 16 of a conventional speculum 12 when the primary blades 14, 16 are in a closed position. Creases 44 run along the length of the blade portion which subdivide the blade portion 36 into a planar spine section 46 and two wing sections 48, 50, each wing section 48, 50 having wing tips 52, 54 corresponding to the maximum width of the blade portion 36. The anchor portion 34 and the spine section 46 of the blade portion 36 lie on substantially the same plane when the supplementary blade 10 is at rest. In this embodiment, the two wing sections 48, 50 are folded about the creases 44 such that the wing tips 52, 54 lie in a common plane parallel to that of the spine section 46, as will be seen clearly from Figures 4, 9 and 10. Alternatively, the blade portion 36 may have only one crease running 44 along its length such that the blade portion 36 is 'V' shaped in cross-section, or equally the blade portion 36 may have no creases and be 'U' shaped in cross-section. The conceptual link here is that the blade portion 36 has at least one wing tip which lies on a different plane from that of the spine section 46 or the anchor portion 34 of the supplemental blade 10.

The blade portion 36 is made of a material, for example a stiff polymer, which is rigid enough to maintain its general shape and contour during use; however, the material may advantageously offer some flexibility and resilience. The hinge portion 38 joins the blade portion 36 to the anchor portion 34 and can be integral with both the blade 36 and anchor portions 34, or separate from them but joined to them. Whilst the anchor portion 34 is attached to the speculum 12 and relatively immobile in relation to the speculum 12, the hinge portion 38 allows the lateral movement of the supplementary blade portion 36 in use. If the supplementary blade 10 is a single piece construction, such as a moulded plastic item, this flexibility at the hinge portion 34 may be achieved by having a reduced thickness at the hinge portion 34, or alternatively forming the hinge portion 34 so that it is adapted to flex, such as a crease or score or a concertina formation. If, alternatively, the supplementary blade 10 is not formed as one-piece, the blade portion 36 and the anchor portion 34 may be formed separately and be joined together such that the join between them forms the hinge portion 34.

The anchor portion 34 comprises an attachment means, such as a fastener 60, for attaching the supplementary blade 10 to the speculum 12. Preferably, the fastener 60 is a pin, stud or toggle which is resiliently press-fitted into the existing joint 20 of the speculum 12 without requiring adaptation of the speculum 12. It will be noted in this respect that the joint 20 of a speculum is commonly a hollow rivet defining a hole that can receive the fastener 60 of the supplementary blade 10. However, alternative fixings such as adhesives will be apparent to the reader. It is also preferred that fixings are releasable so that if the physician decides not to use the supplementary blades for any reason, they can be removed and discarded.

In use, the supplementary blade 10 is positioned on and attached to a joint 20 of the speculum hinge 18 by its fastener 60 so that the inside face 30 of the supplementary blade 10 faces and embraces the outside faces of the primary blades 14, 16 of the speculum. It is envisaged that two supplementary blades 10 of the present invention will be used with a speculum 12, each blade 10 being attached to one of the two hinge joints 20, on either side of the speculum 12.

When the primary blades 14, 16 of the speculum 12 are in the closed position, the supplementary blades 10 lie close enough to the primary blades 14, 16 that the speculum 12 with its supplementary blades 10 can be inserted smoothly into the vaginal cavity. It will be noted in particular that the distal region 42 of each supplementary blade 10 is in contact with, or lies close to, the primary blades 14, 16 when the primary blades 14, 16 are in the closed position.

As can be seen most clearly from Figure 2, the fastener 60 has a double-waisted cross-section shape to provide two positions of attachment of the supplementary blade 10 relative to the speculum 12, marked A and B. It will be appreciated that when the supplementary blade 10 is attached to the speculum 12 in the first position A, the anchor portion 34 and hence the proximal end 40 of the blade portion 36 of the supplementary blade 10 is spaced further apart from the primary blades 14, 16 of the speculum 12 than when the attachment is in the second position B. The two attachment positions A, B provide a different supplemental blade splaying effect, which will be described below with reference to Figures 5 to 10.

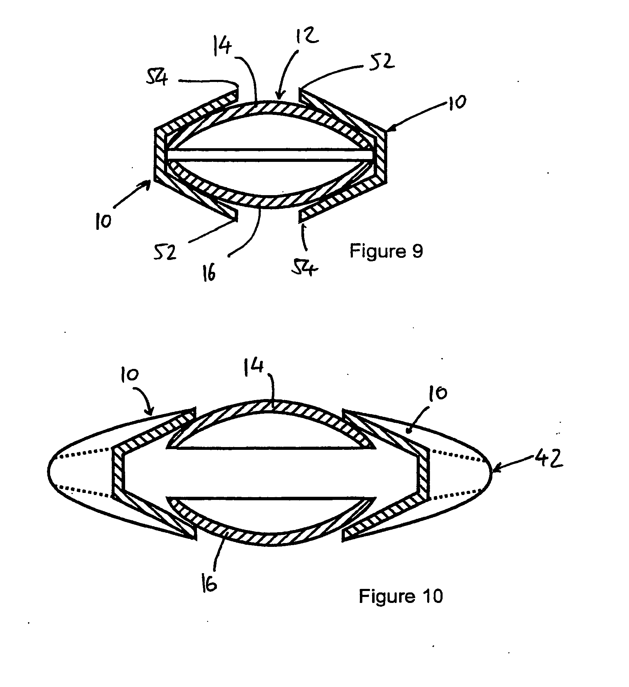

Referring now to Figures 5 to 10, in use, as the primary blades 14, 16 of the speculum 12 are opened or splayed in conventional manner, the supplementary blade 10 attached to the speculum 12 is caused to splay outwards away from the body of the speculum 12 through interaction with at least one (and in this embodiment, both) of the primary blades 14, 16. When inside the vaginal cavity, the outward splaying of the supplementary blade 10 pushes back the vaginal side walls affording the physician unhindered access to the vaginal canal for examination or treatment. Initially, when the primary blades 14, 16 are closed, at least the distal region 42 of the blade portion 36 of the supplementary blade 10 is in contact with at least one primary blade. As the primary blades 14, 16 are opened further apart and the supplementary blade 10 splays further outward, the leading area of contact on the blade portion 36 moves proximally, until it reaches or approaches the wing tip 52, 54, at which point the supplementary blade portion 10 is at its maximum splay position. This can be most clearly seen in Figure 10.

When the supplementary blade 10 is attached to the speculum 12 at position A of the fastener 60, the proximal end 40 of the supplementary blade 10 with the associated wing tips 52, 54 is spaced further away from the primary blades 14, 16 of the speculum 12. This means that the supplementary blade 10 is caused to splay away from the body of the speculum 12 later in the opening movement, i.e. when the primary blades 14, 16 are open further, compared with attachment position B. In other words, attachment position A is chosen when a milder lateral splay effect is required.

Although not shown, the present invention also includes a further embodiment of the supplementary blade 10 of Figure 1 wherein the anchor portion 34, the blade portion 36 and the hinge portion 38 are substantially immobile in relation to each other.

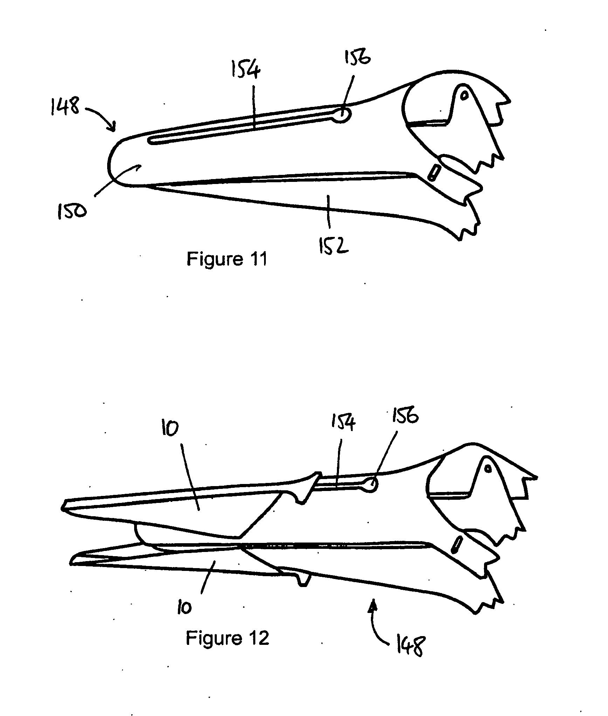

Figure 11 shows an alternative speculum 148 which differs from the conventional speculum 12 of Figures 5 to 10 in that it includes an upper blade 150 and a lower blade 152, each of which include a substantially rectangular slot, channel or guiding means 154 extending between the distal and proximal ends of the blades 150, 152. Each slot 154 is centrally positioned with respect to its associated blade 150, 152 and terminates at its proximal end with an opening 156 slightly wider than the width of the slot 154.

As can most clearly be seen in Figure 12, the speculum 148 is intended to be assembled with a supplementary blade, such as the supplementary blade 10 of Figure 1, to increase the functional length of the speculum 148. The proximal opening 156 of the slot 154 is shaped to receive the fastener 60 of the supplementary blade 10 by press-fitting. Once assembled, the supplementary blade 10 can be slid along the length of the slot 154 to vary the functional length of the speculum 148.

Although Figures 11 and 12 show the speculum 148 including a slot 154 on both its upper and lower blades 150, 152, it is also possible to provide a slot 154 on only one of the blades 150, 152 so that the length of the speculum 148 is variable on only one side. Similarly, where slots 154 are provided on both of the upper and lower blades 150, 152, their supplementary blades 10 can be extended to different functional lengths.

In another arrangement, not shown, it is possible to replace the slots 154 with a linear array of holes spaced along the length of the upper and/or lower blades 150, 152. This provides a plurality of selectable anchorage points to choose for various functional lengths.

Also contemplated is a conventional speculum 12 adapted to include one or more of the slots 154 described above on its upper and/or lower blades 14, 16.

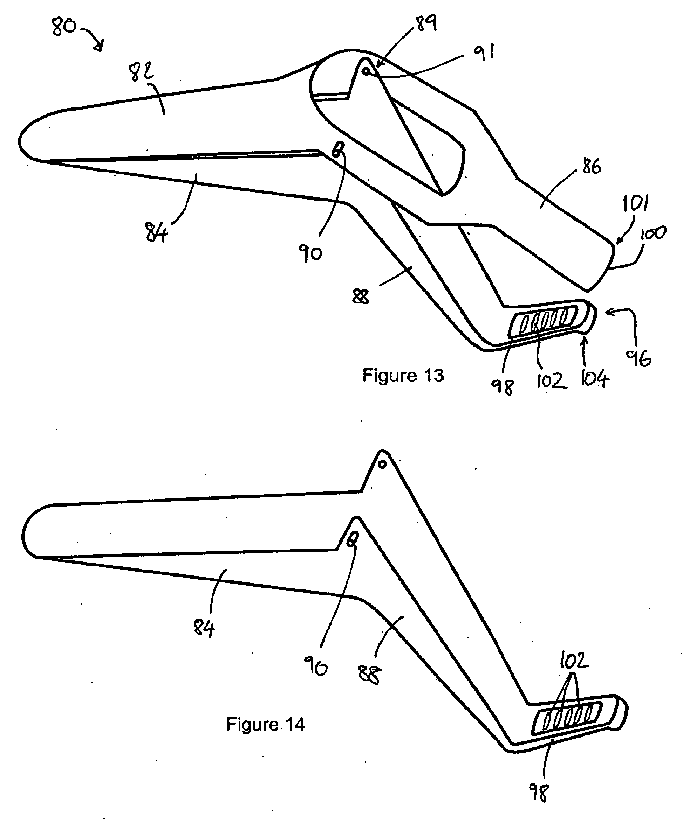

Referring now to Figures 13 to 16, it is also contemplated a speculum 80 having a simplified locking mechanism for locking the position of the open blades. The speculum 80 comprises an upper blade 82 and a lower blade 84 of substantially conventional shape, the upper and lower blades 82, 84 having associated upper and lower handles 86, 88, respectively. The upper and lower blades 82, 84 are connectable to each other at their proximal ends by a hinge 89, the hinge 89 comprising two joints 91, one on each side of the blades 82, 84. Each joint 91 consists of a pin 90 formed at, or joined to, the proximal end of the lower blade 84 (as can most clearly be seen in Figure 14) and an opening 92 for receiving the pin 90, at the proximal end of the upper blade 82. Alternatively, the pin 90 may be formed at, or joined to the upper blade 82, and the opening on the lower blade 84. As before, the proximal ends of the upper and lower blades 82, 84 define a proximal aperture, through which the vaginal canal can be accessed in use.

Both the upper and lower handles 86, 88 depend from the proximal ends of the upper and lower blades 82, 84, respectively, at an obtuse angle to the blades 82, 84. In other words, the upper and lower handles 86, 88 depend rearwardly or proximally from the blades 82, 84. The angle of the upper handle 86 from the upper blade 82 is less than the angle of the lower handle 88 from the lower blade 84. Squeezing the handles 86, 88 together, or pressing the upper handle 86 distally towards the lower handle 88 flares the blades 82, 84 apart at their distal ends. In conventional manner, the separation of the blades 82, 84 is greatest at their distal ends and decreases towards their proximal ends.

Conveniently, in use, the position of the flared blades 82, 84 can be held or locked in position against inward pressure from the vaginal walls by a ratchet means 96 which comprises a ratchet portion 98 on the lower handle 88, and an edge 100 at the free end 101 of the upper handle 86 co-operable with the ratchet portion 98. The ratchet portion 98 comprises a series of protrusions 102 or teeth and is at the free end 104 of the lower handle, depending orthogonally rearwardly from the handle 88. An open blade position is held or locked by engaging or abutting the edge 100 at the free end of the upper handle 86 against one of the protrusions 102, by virtue of the open blades 82, 84 being in compression from contraction of the vaginal wall muscles.

The upper and lower blades 82, 84 can be easily disconnected from each other by virtue of the pin and hole arrangement of the joint 91 and some flexibility of the material from which the blades are made. This provides the physician with greater flexibility during examination and treatment, such as when better access is required through the introitus of the vagina to accommodate certain medical instruments. In this case the lower blade 84 and upper blade 82 are detached or disassembled from each other and either one or both of the blades are used as a retractor or retractors.

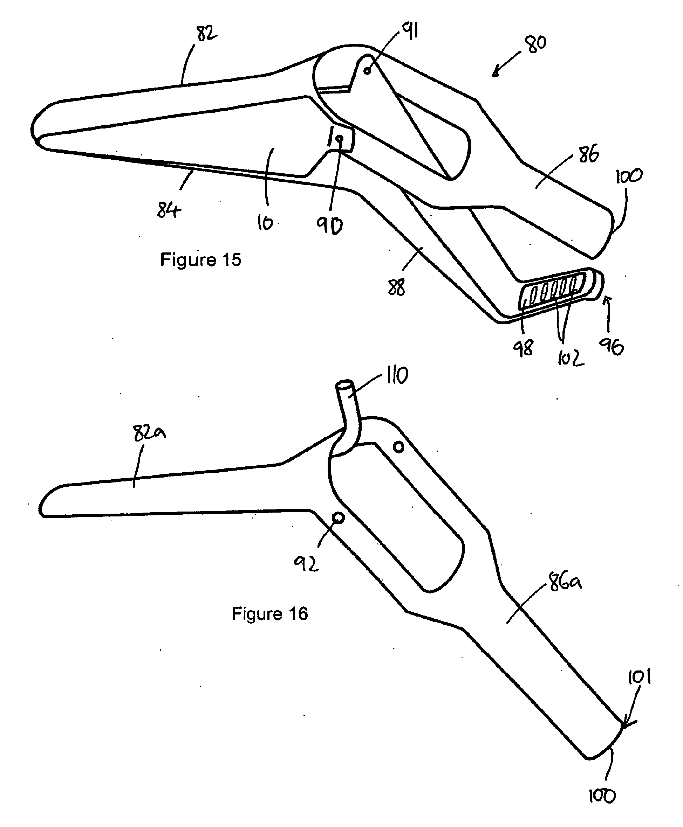

Figure 15 shows the speculum 80 of Figure 13 assembled with the supplementary blade 10 featured in Figures 1 to 10. Operation of this speculum 80 results in lateral splaying of the supplementary blade 10 through co-operation with at least one of the speculum blades, as described above with reference to Figures 1 to 10.

Figure 16 shows an alternative upper blade 82a and handle 86a construction to that shown in Figures 13 and 15, having a smoke extraction tube 110 incorporated into the proximal end of the upper blade 82a.

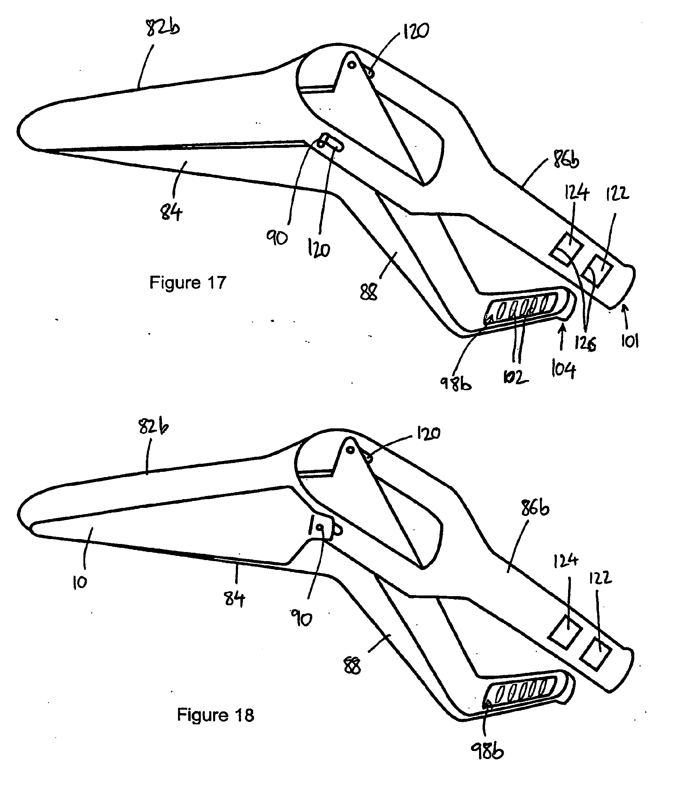

Figures 17 to 19 show alternative arrangements of the specula of Figures 13 to 16, having a further alternative upper blade 82b and handle 86b construction, which allows increased dilation of the introitus of the vagina without disassembling. The hinge between the upper blade 82b and the lower blade 84 is a mobile or floating hinge , defined by each joint comprising an elongated opening or slot 120 on the upper blade 82b for receiving the pin 90 of the lower blade 84. It will be appreciated that the proximal opening is enlarged by moving the lower blade 84 and upper blade 82b relative to each other so that the pin 90 lies towards the handle end of the slot 120. The ratchet means of this alternative arrangement comprises a ratchet portion 98b at the free end 104 of the lower handle, as before, and two openings 122, 124 in the free end of the upper handle 86b which are wide enough to receive the ratchet portion 98b. In use, the flared apart position of the blades can be held or locked in place by the engagement of a protrusion 102 of the ratchet means 96b with an edge 126 of one of the openings 124. The lower opening 122 is selected when an enlarged proximal opening is required; conversely the upper opening 124 is selected when a smaller proximal opening is required. The upper and lower blades may also be detached or disassembled from each other, enabling one or both of the blades to be used as a retractor or retractors, and for ease of cleaning.

Figure 18 shows the speculum of Figure 17 assembled with the supplementary blade of Figures 1 to 10.

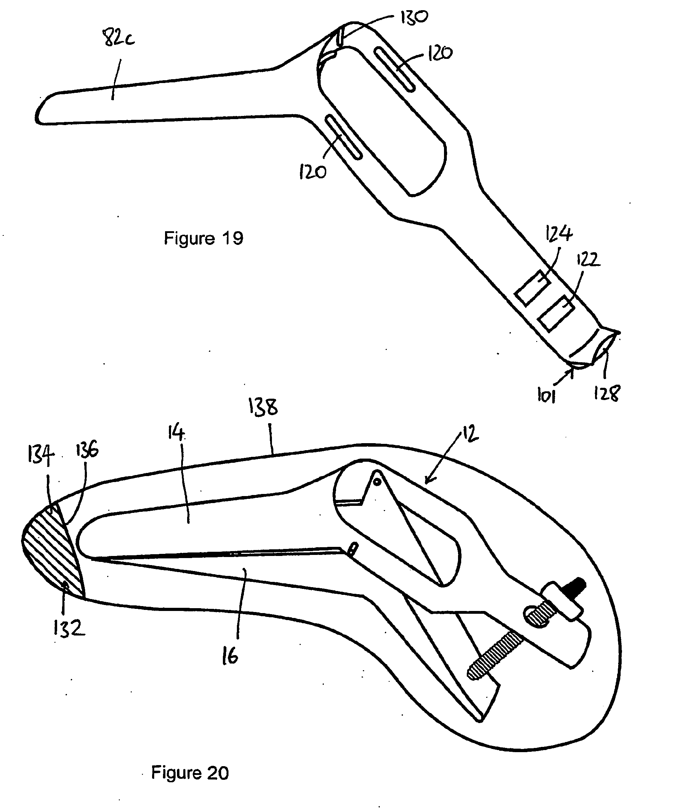

A variant of the upper blade of this alternative arrangement is shown in Figure 19, having a thumb support 128 at the free end 101 of the upper handle, and clips 130 at the proximal end of the upper blade 82c for holding a smoke extraction tube which may be inserted into the vaginal cavity during electrosurgery, for example. The clips 130 may be separate parts attached to the upper blade 82c of the speculum but are preferably integral with the upper blade 82c, being tabs cut out and bent away from that blade. The construction of the speculum and thumb support 128 helps a physician to adjust the openings of the blades and lock them in position relative to each other using one hand.

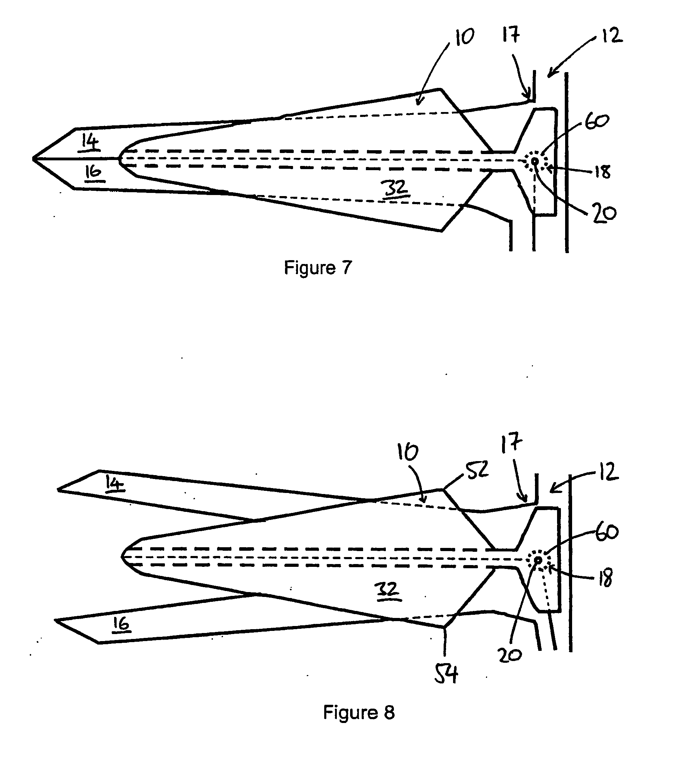

In Figure 20 is shown the conventional speculum 12 of Figures 5 and 8 with two primary blades 14, 16 and having a lubrication means for lubricating the blades 14, 16 of the conventional speculum 12 for ease of insertion of the blades 14, 16 into the vaginal cavity of a patient. The lubrication means comprises a blister 132 containing a lubricant 134, such as KY Jelly (trade mark) or any other suitable lubricant, constrained by a thin membrane 136. The blister 132 is on the inside of a package 138 containing the sterile conventional speculum 12.

In use, a practitioner bursts the blister 132 of lubricant 134 to smear the lubricant 134 on the tips of the blades 14, 16 of the conventional speculum 12 by applying pressure on the membrane 136 with the blade tips, before opening the package 138. In this way, the practitioner avoids having to use his or her fingers to smear lubricant on the conventional speculum 12, and the conventional speculum 12 is lubricated while still within its sterile packaging. Although not shown, the lubrication means can also be used with any of the specula of Figures 7 to 19. Also, the blister 132 may contain medicinal or therapeutic products such as antibiotic creams or lotions instead of, or as well as, the lubricant 134.

Also contemplated is an alternative lubrication means for specula, which comprises a pre-lubricated speculum sealed within the package 138. As before, a medicinal or therapeutic product may also or alternatively be applied to the speculum thus packaged.

## Claims

1. A supplementary blade for a speculum, the speculum (12) having a plurality of primary blades (14, 16) movable relative to each other for opening and closing, wherein the supplementary blade (10) comprises an anchor portion (34) attachable to the speculum (12) and a blade portion (36) movable with respect to the anchor portion (34), and the blade portion (36) is shaped to interact with at least one of the primary blades (14, 16) to move into an extended position in response to opening of the primary blades (14,16), the supplementary blade (10) having a concave inner face (30) shaped to lie against a convex outer surface of one or more of the primary blades (14,16).

2. The blade of Claim 1 and being resilient to return to a retracted position when the primary blades (14, 16) close.

3. The blade of Claim 1 or Claim 2 and having at least one wing (48, 50) lying outside a plane containing the anchor portion (34).

4. The blade of any of Claims 1 to 3 and including a spine portion (46) extending from the anchor portion (34).

5. The blade of any of Claims 1 to 4, and having a continuously curved lateral cross-section.

6. The blade of any of Claims 1 to 4, and having a discontinuous lateral cross section interrupted by at least one crease (44).

7. The blade of Claim 6, wherein there are parallel creases (44).

8. The blade of Claim 6 or Claim 7, wherein the cross-section includes two or more generally flat portions extending from the or each crease (44).

9. The blade of any preceding Claim, and having a major portion tapering generally toward a free end opposed to the anchor portion (34).

10. The blade of Claim 9, and having a minor portion tapering generally toward the anchor portion (34).

11. The blade of Claim 9, wherein a neck joins the anchor portion (34) to the blade portion (36).

12. The blade of any preceding Claim, wherein the anchor portion (34) comprises a releasable fastening (6) attachable to a speculum (12).

13. The blade of any preceding Claim, wherein the anchor portion (34) comprises a fastening (60) attachable to a speculum (12) in two or more attachment positions (A, B).

14. The blade of Claim 13, wherein a first attachment position (A) is laterally inward of a second attachment position (B).

15. The blade of any of Claims 12 to 14, wherein the fastening (60) comprises a stud engageable within a hole in the speculum.

16. The blade of Claim 15 when appendant to Claim 13 or Claim 14, wherein the stud has two or more waists each defining a respective attachment position.

17. The blade of any preceding Claim, whose lateral cross-section comprises a spine (46) and two wings (48, 50) lying to the same side of the spine.

18. The blade of Claim 17, whose lateral cross-section is symmetrical about the central longitudinal axis of the spine.

19. A speculum (12) having a plurality of primary blades (14, 16) movable relative to each other for opening and closing, and being fitted with at least one supplementary blade (10) according to any preceding Claim, the supplementary blade (10) being positioned to interact with at least one of the primary blades to move into an extended position in response to opening of the primary blades.

20. The speculum of Claim 19, and having a means for attachment that is adapted to attach the supplementary blade (10) to the speculum (12) in a releasable manner.

## Patentansprüche

1. Zusatzblatt für ein Spekulum, wobei das Spekulum (12) mehrere zum Öffnen und Schließen relativ zueinander bewegbare Primärblätter (14, 16) aufweist, wobei das Zusatzblatt (10) einen an dem Spekulum (12) befestigbaren Ankerabschnitt (34) und einen gegenüber dem Ankerabschnitt (34) bewegbaren Blattabschnitt (36) umfasst, und der Blattabschnitt (36) dazu geformt ist, mit mindestens einem der Primärblätter (14, 16) in Wechselwirkung zu stehen, um sich als Reaktion auf Öffnen der Primärblätter (14, 16) in eine ausgeklappte Position zu bewegen, wobei das Zusatzblatt (10) eine konkave Innenseite (30) aufweist, die dazu geformt ist, an einer konkaven Außenfläche von einer oder mehreren der Primärblätter (14,16) anzuliegen.

2. Blatt nach Anspruch 1, das elastisch ist, um zu einer eingeklappten Position zurückzukehren, wenn die Primärblätter (14, 16) schließen.

3. Blatt nach Anspruch 1 oder Anspruch 2 mit mindestens einem Flügel (48, 50), der außerhalb einer den Ankerabschnitt (34) enthaltenden Ebene liegt.

4. Blatt nach einem der Ansprüche 1 bis 3, umfassend einen Rückenabschnitt (46), der sich von dem Ankerabschnitt (34) erstreckt.

5. Blatt nach einem der Ansprüche 1 bis 4 mit einem kontinuierlich gekrümmten Querschnitt.

6. Blatt nach einem der Ansprüche 1 bis 4 mit einem diskontinuierlichen Querschnitt, der durch mindestens einen Knick (44) unterbrochen ist.

7. Blatt nach Anspruch 6, wobei parallele Knicke (44) vorliegen.

8. Blatt nach Anspruch 6 oder Anspruch 7, wobei der Querschnitt zwei oder mehr allgemein flache Abschnitte umfasst, die sich von dem bzw. jedem Knick (44) erstrecken.

9. Blatt nach einem der vorangehenden Ansprüche mit einem Hauptabschnitt, der allgemein zu einem dem Ankerabschnitt (34) gegenüberliegenden freien Ende hin verjüngt ist.

10. Blatt nach Anspruch 9 mit einem Nebenabschnitt, der allgemein zu dem Ankerabschnitt (34) hin verjüngt ist.

11. Blatt nach Anspruch 9, wobei ein Hals den Ankerabschnitt (34) mit dem Blattabschnitt (36) verbindet.

12. Blatt nach einem der vorangehenden Ansprüche, wobei der Ankerabschnitt (34) eine lösbare Befestigungsvorrichtung (6) umfasst, die an einem Spekulum (12) befestigbar ist.

13. Blatt nach einem der vorangehenden Ansprüche, wobei der Ankerabschnitt (34) eine Befestigungsvorrichtung (60) umfasst, die an zwei oder mehr Befestigungspositionen (A, B) an einem Spekulum (13) befestigbar ist.

14. Blatt nach Anspruch 13, wobei eine erste Befestigungsposition (A) seitlich innerhalb von einer zweiten Befestigungsposition (B) liegt.

15. Blatt nach einem der Ansprüche 12 bis 14, wobei die Befestigungsvorrichtung (60) einen in einem Loch in dem Spekulum einrastbaren Zapfen umfasst.

16. Blatt nach Anspruch 15 wenn zugehörig zu Anspruch 13 oder Anspruch 14, wobei der Zapfen zwei oder mehr Einschnürungen umfasst, die jeweils eine entsprechende Befestigungsposition definieren.

17. Blatt nach einem der vorangehenden Ansprüche, dessen Querschnitt einen Rücken (46) und zwei auf der selben Seite des Rückens liegende Flügel (48, 50) umfasst.

18. Blatt nach Anspruch 17, dessen Querschnitt um die mittlere Längsachse des Rückens symmetrisch ist.

19. Spekulum (12) mit mehreren zum Öffnen und Schließen relativ zueinander bewegbaren Primärblättern (14, 16), das mit mindestens einem Zusatzblatt (10) nach einem der vorangehenden Ansprüche ausgestattet ist, wobei das Zusatzblatt (10) dazu positioniert ist, mit mindestens einem der Primärblätter in Wechselwirkung zu stehen, um sich als Reaktion auf das Öffnen der Primärblätter in eine ausgeklappte Position zu bewegen.

20. Spekulum nach Anspruch 19 mit einem Mittel zum Befestigen, das dazu angepasst ist, das Zusatzblatt (10) auf lösbare Weise an dem Spekulum (12) zu befestigen.

## Revendications

1. Valve supplémentaire pour un spéculum, le spéculum (12) étant pourvu d'une pluralité de valves (14, 16) primaires mobiles les unes par rapport aux autres pour s'ouvrir et se refermer, dans laquelle la valve (10) supplémentaire comporte une partie (34) ancrage pouvant être fixée au spéculum (12) et une partie (36) valve mobile par rapport à la partie (34) ancrage, et la partie (36) valve est formée pour une interaction avec au moins une des valves (14, 16) primaires afin de se déplacer dans une position sortie en réponse à l'ouverture des valves (14, 16) primaires, la valve (10) supplémentaire présentant une face (30) interne concave formée pour reposer contre une surface externe convexe de l'une ou plusieurs des valves (14, 16) primaires.

2. Valve selon la revendication 1 et qui est résiliente pour revenir à une position rentrée lorsque les valves (14, 16) primaires se referment.

3. Valve selon la revendication 1 ou la revendication 2 et qui est pourvue d'au moins une ailette (48, 50) reposant à l'extérieur d'un plan contenant la partie (34) ancrage.

4. Valve selon l'une quelconque des revendications 1 à 3 et qui comprend une partie (46) arête dorsale s'étendant depuis la partie (34) ancrage.

5. Valve selon l'une des revendications 1 à 4, et qui présente une section transversale latérale continuellement courbe.

6. Valve selon l'une quelconque des revendications 1 à 4, et qui présente une section transversale latérale discontinue interrompue par au moins un pli (44).

7. Valve selon la revendication 6, dans laquelle il y a des plis (44) parallèles.

8. Valve selon la revendication 6 ou la revendication 7, dans laquelle la section transversale comprend deux parties globalement plates ou plus s'étendant depuis ledit ou chaque pli (44).

9. Valve selon l'une quelconque des revendications précédentes, et qui présente une plus grande partie s'amincissant globalement vers une extrémité libre opposée à la partie (34) ancrage.

10. Valve selon la revendication 9, et qui présente une plus petite partie s'amincissant globalement vers la partie (34) ancrage.

11. Valve selon la revendication 9, dans laquelle un col joint la partie (34) ancrage à la partie (36) valve.

12. Valve selon l'une quelconque des revendications précédentes, dans laquelle la partie (34) ancrage comporte une attache (6) libérable pouvant être fixée à un spéculum (12).

13. Valve selon l'une quelconque des revendications précédentes, dans laquelle la partie (34) ancrage comporte une attache (60) pouvant être fixée à un spéculum (12) dans deux positions (A, B) de fixation ou plus.

14. Valve selon la revendication 13, dans laquelle une première position (A) de fixation est latéralement vers l'intérieur d'une seconde position (B) de fixation.

15. Valve selon l'une quelconque des revendications 12 à 14, dans laquelle l'attache (60) comporte un goujon pouvant être mis en prise à l'intérieur d'un trou dans le spéculum.

16. Valve selon la revendication 15 lorsqu'elle est annexée à la revendication 13 ou la revendication 14, dans laquelle le goujon présente deux dégagements ou plus, chacun délimitant une position de fixation respective.

17. Valve selon l'une quelconque des revendications précédentes, dont la section transversale latérale comporte une arête dorsale (46) et deux ailettes (48, 50) reposant du même côté que l'arête dorsale.

18. Valve selon la revendication 17, dont la section transversale latérale est symétrique autour de l'axe central longitudinal de l'arête dorsale.

19. Spéculum (12) étant pourvu d'une pluralité de valves (14, 16) primaires mobiles les unes par rapport aux autres pour s'ouvrir et se refermer, et étant monté avec au moins une valve (10) supplémentaire selon l'une quelconque des revendications précédentes, la valve (10) supplémentaire étant positionnée pour une interaction avec au moins une des valves primaires afin de se déplacer dans une position sortie en réponse à l'ouverture des valves primaires.

20. Spéculum selon la revendication 19, et étant pourvu d'un moyen de fixation qui est conçu pour fixer la valve (10) supplémentaire au spéculum (12) d'une manière libérable.
